# EUROPÄISCHE PATENTSCHRIFT

(11) **EP 1 977 680 B1**
(45) Veröffentlichungstag und Bekanntmachung des Hinweises auf die Patenterteilung: **02.06.2010**
(21) Anmeldenummer: 08152395.3
(22) Anmeldetag: 06.03.2008
(51) Int. Cl.: A61B 1/015, A61B 1/012

(54) **Druckmesseinrichtung**
Pressure gauge device
Dispositif de mesure de la pression

(30) Priorität: 03.04.2007 DE 102007000200
(43) Veröffentlichungstag der Anmeldung: 08.10.2008
(73) Patentinhaber: INVENDO MEDICAL GMBH, 69469 Weinheim (DE)
(72) Erfinder: Viebach, Thomas, 86579 Diepoltshofen (DE)
(74) Vertreter: Winter, Brandl, Fürniss, Hübner Röss, Kaiser, Polte Partnerschaft Patent- und Rechtsanwaltskanzlei

(56) Entgegenhaltungen:
- EP-A- 1 213 035
- EP-A- 1 253 412
- DE-A1- 2 823 670
- DE-U1- 20 206 474
- US-A- 3 418 853
- US-A- 3 795 140
- US-A- 5 000 049
- US-A- 5 756 900

## Beschreibung

Die vorliegende Erfindung bezieht sich auf eine Druckmesseinrichtung und im Speziellen auf eine Druckmesseinrichtung zur Messung eines Fluiddrucks.

Endoskopievorrichtungen sowie Vorrichtungen zum Einführen eines medizinischen Endoskops in einen Körperkanal werden beispielsweise in der DE 39 25 484 A1 beschrieben. Die darin beschriebenen Vorrichtungen erlauben es, dass ein Endoskop nicht mehr in den zu untersuchenden Körper hineingeschoben wird, sondern sich selber hineinbewegt. Zu diesem Zweck ist das Endoskop mit einem Eigenantrieb ausgestattet, der ein unkomplizierteres und schnelleres Einführen ermöglicht.

Als solch ein Eigenantrieb kann beispielsweise auch ein so genannter Stülpschlauch verwendet werden, in den der Endoskopschaft eingeführt ist. Beim Vortrieb des Endoskops treten unterschiedliche Relativbewegungen auf. Zum einen tritt eine Relativbewegung zwischen dem Endoskopschaft und dem Stülpschlauch auf, die miteinander in Gleitkontakt stehen. Zum anderen tritt auch eine Relativbewegung zwischen einem innenliegenden und einem außenliegenden Abschnitt des sich abwickelnden Stülpschlauchs auf.

Um die jeweils auftretende Gleitreibung sowie eine Haftreibung zwischen jeweiligen Elementen zu verringern, ist beispielsweise der Einsatz eines von Außen zugeführten Schmiermittels vorgeschlagen worden, beispielsweise in der EP-A-0 873 761. In beiden Fällen wird zwischen den Endoskopschaft und den Stülpschlauch einerseits sowie zwischen die beiden übereinander zu liegen kommenden Stülpschlauchabschnitte andererseits ein Schmiermittel zugeführt, das in einem Fluidbeutel bevorratet sein kann. Um den Druck des zu der Schmierstelle zwischen Endoskopschaft und Stülpschlauch zugeführten Schmiermittels zu kontrollieren, muss dieser von einer Druckmesseinrichtung erfasst werden, die zwischen dem Hydraulikfluidbeutel und der Schmierstelle zwischen Endoskopschaft angeordnet ist.

Wenn die Endoskopvorrichtung als Einwegvorrichtung ausgebildet ist, werden nach Gebrauch alle Elemente der Endoskopvorrichtung weggeworfen, die mit Körperflüssigkeit des untersuchten Patienten in Kontakt gekommen sein könnten, wie der Endoskopschaft samt Stülpschlauch, der Fluidbeutel und die Druckmesseinrichtung.

Dokument US 3 418 853 offenbart eine Druckmesseinrichtung.

Daher ist es die Aufgabe der Erfindung eine einfache und kostengünstige Druckmesseinrichtung vorzusehen, die einer Einwegkonstruktion von beispielsweise einer Endoskopvorrichtung Rechnung trägt.

Die Aufgabe der Erfindung wird mit einer Druckmesseinrichtung mit den Merkmalen gemäß dem Patentanspruch 1 gelöst.

Vorteilhafte Weiterbildungen der Erfindung sind Gegenstand der Unteransprüche.

Der Kern der Erfindung besteht demzufolge darin, dass die Druckmesseinrichtung aus zwei getrennten Bauteilen besteht, die zusammensteckbar und zerstörungsfrei wieder lösbar sind. Das eine Bauteil ist ein vorzugsweise kostengünstig herstellbares Druck-Bewegungsumwandlungselement, das beweglichen Abschnitt hat, der einen Druck aufnimmt und in dessen Abhängigkeit eine Bewegung ausführt und das andere Bauteil ist ein Kraftmesselement, welches eine Kraft misst, die durch die Bewegung des beweglichen Abschnitts auf das Kraftmesselement ausgeübt wird. Die Bauteile sind so konstruiert, dass bei deren funktionaler Kopplung nur das Druck-Bewegungsumwandlungselement mit dem Druckmedium isoliert bleibt. Daher kann das kostengünstig herstellbare Druck-Bewegungsumwandlungselement nach einmaligem Gebrauch weggeschmissen werden, wohingegen das Kraftmesselement, welches in der Regel teurer ist, wieder verwendet werden kann. Durch diese konstruktive sowie elementare Aufteilung der Druckmesseinrichtung in ein kostengünstiges Einwegbauteil und ein wieder verwendbares Bauteil, ist es bei Einweglösungen nicht erforderlich, die gesamte Druckmesseinrichtung wegzuwerfen oder unter Zeitaufwand zu demontieren und zu reinigen, sondern es kann nur das kostengünstig herstellbare Bauteil weggeworfen werden, während das andere Bauteil wieder verwendet werden kann.

Wird die Druckmesseinrichtung gemäß der vorliegenden Erfindung auf eine Einwegendoskopvorrichtung angewendet, bei der Endoskopschaft, Stülpschlauch sowie ein durch Schläuche mit dem Endoskop fluidverbundener Fluidbeutel bzw. -behälter, der ein Schmiermittel für eine Schmierung zwischen Endoskopschaft und Stülpschlauch sowie im Inneren des Stülpschlauchs bevorratet, nach einmaligem Gebrauch weggeworfen werden, kann das Druck-Bewegungsumwandlungselement, das als Einwegartikel konstruiert ist, nach Gebrauch von dem Kraftmesselement getrennt und weggeworfen werden. Das Kraftmesselement hingegen kann wieder verwendet werden. Auf diese Weise kann eine kostengünstige Einwegendoskopvorrichtung realisiert werden.

Vorzugsweise ist das Druck-Bewegungsumwandlungselement als eine gas- bzw. fluiddichte Einheit ausgebildet, um ein Austreten von Gas bzw. Fluid in die Umgebung zu verhindern, wie es besonders bei Endoskopanwendungen erfordert ist.

Das Druck-Bewegungsumwandlungselement hat bevorzugt einen Aufnahmeabschnitt, der einen Abschnitt eines Behältnisses oder Schlauchs aufnimmt, um den Druck eines Fluids oder Gases zu erfassen, das im Inneren des Behältnisses oder Schlauchs strömt.

In einer vorteilhaften Ausführungsform der Erfindung nimmt der Aufnahmeabschnitt einen Schlauchabschnitt eines Schlauchs auf, der mit einem Einwegfluidbeutel verbunden ist, welcher ein Fluid bevorratet. Bevorzugt wird der Schlauchabschnitt selbst zur Ausbildung einer fluiddichten Verbindung zwischen sich selbst und dem Aufnahmeabschnitt verwendet.

Bevorzugt ist der bewegliche Abschnitt des Druck-Bewegungsumwandlungselement ein Kolben oder eine Membran. Es kann aber jedes beliebige Element andere Element sein, welches sich bei Änderung eines einwirkenden Drucks bewegt, wie beispielsweise ein gelenkig gelagerter Hebel, etc. Die Bewegung des beweglichen Abschnitts ist nicht auf eine translatorische Bewegung beschränkt, sondern kann eine beliebige Bewegung, wie Translation, Rotation oder eine Kombination aus Translation und Rotation sein.

Bevorzugt ist das Kraftmesselement ein Kraftmesssensor, der die gemessene Kraft in elektrische Signale umwandelt, wie z.B. ein Piezoelement. Das Kraftmesselement kann aber auch nach einem anderen Prinzip funktionieren, beispielsweise rein mechanisch, solange gewährleistet ist, dass von der durch das Kraftmesselement gemessenen Kraft auf den Druck zurückgeschlossen werden kann, mit dem das Druck-Bewegungsumwandlungselement beaufschlagt wird.

Vorteilhafterweise ist das Kraftmesselement oder das Druck-Bewegungsumwandlungselement mit einer Positionierungseinrichtung versehen, um das Druck-Bewegungsumwandlungselement in dem zusammengesteckten Zustand relativ zu dem Kraftmesselement in einer vorbestimmten Lage zu positionieren.

Darüber hinaus kann das Kraftmesselement oder das Druck-Bewegungsumwandlungselement vorteilhafterweise mit einer Arretierungseinrichtung versehen sein, um das Druck-Bewegungsumwandlungselement in dem zusammengesteckten Zustand relativ zu dem Kraftmesselement zu fixieren.

Die Erfindung wird nunmehr nachfolgend anhand eines bevorzugten Ausführungsbeispiels unter Bezugnahme auf die begleitenden Zeichnungen näher erläutert.

Fig. 1a ist eine perspektivische Ansicht eines Fluidbeutels mit Verbindungsschläuchen, von denen einer mit einem Druck-Bewegungsumwandlungselement gemäß der Erfindung versehen ist.

Fig. 1b ist eine Seitenansicht des Fluidbeutels, der in Fig. 1a dargestellt ist.

Fig. 1c ist eine vergrößerte Darstellung des Ausschnitts A von Fig. 1b.

Fig. 1d ist eine Rückansicht des Fluidbeutels, der in Fig. 1a dargestellt ist.

Fig. 2 ist eine Explosionsansicht eines Druck-Bewegungsumwandlungselements gemäß einer ersten Ausführungsform der Erfindung.

Fig. 3 ist eine Perspektivenansicht des Druck-Bewegungsumwandlungselements gemäß der ersten Ausführungsform der Erfindung.

Fig. 4a ist eine Ansicht von oben des Druck-Bewegungsumwandlungselements gemäß der ersten Ausführungsform der Erfindung.

Fig. 4b ist eine Seitenansicht des Druck-Bewegungsumwandlungselements gemäß der ersten Ausführungsform der Erfindung.

Fig. 4c ist eine Draufsicht des Druck-Bewegungsumwandlungselements gemäß der ersten Ausführungsform der Erfindung.

Fig. 4d ist eine Schnittansicht des Druck-Bewegungsumwandlungselements gemäß der ersten Ausführungsform der Erfindung entlang der Linie A-A in Fig. 4c.

Fig. 4e ist eine Schnittansicht des Druck-Bewegungsumwandlungselements gemäß der ersten Ausführungsform der Erfindung entlang der Linie B-B in Fig. 4c.

Fig. 5 ist eine Explosionsansicht des Kraftmesselements gemäß der Erfindung.

Fig. 6a und 6b sind Perspektivenansichten des Kraftmesselements gemäß der Erfindung.

Fig. 7a ist eine Draufsicht des Kraftmesselements gemäß der Erfindung.

Fig. 7b und 7c sind Seitenansichten des Kraftmesselements gemäß der Erfindung.

Fig. 7d ist eine Ansicht von oben des Kraftmesselements gemäß der Erfindung.

Fig. 7e ist eine Schnittansicht des Kraftmesselements gemäß der Erfindung entlang Linie A-A in Fig. 7d.

Fig. 8a ist eine Perspektivenansicht der Druckmesseinrichtung gemäß der vorliegenden Erfindung in nicht zusammengestecktem Zustand.

Fig. 8b ist eine Perspektivenansicht der Druckmesseinrichtung gemäß der vorliegenden Erfindung in zusammengestecktem Zustand.

Fig. 9a ist eine Rückansicht der Druckmesseinrichtung gemäß der vorliegenden Erfindung in zusammengestecktem Zustand.

Fig. 9b ist eine Ansicht von oben auf die Druckmesseinrichtung gemäß der vorliegenden Erfindung in zusammengestecktem Zustand.

Fig. 9c ist eine Seitenansicht der Druckmesseinrichtung gemäß der vorliegenden Erfindung in zusammengestecktem Zustand.

Fig. 9d ist eine Vorderansicht der Druckmesseinrichtung gemäß der vorliegenden Erfindung in zusammengestecktem Zustand.

Fig. 9e ist eine Schnittansicht der Druckmesseinrichtung gemäß der vorliegenden Erfindung in zusammengestecktem Zustand entlang der Linie B-B in Fig. 9c.

Fig. 9f ist eine Schnittansicht der Druckmesseinrichtung gemäß der vorliegenden Erfindung in zusammengestecktem Zustand entlang der Linie A-A in Fig. 9d.

Fig. 10 ist eine Perspektivenansicht des Druck-Bewegungsumwandlungselements gemäß einer zweiten Ausführungsform der Erfindung.

Fig. 11a ist eine Rückansicht des Druck-Bewegungsumwandlungselements gemäß einer zweiten Ausführungsform der Erfindung.

Fig. 11b ist eine Ansicht von oben auf das Druck-Bewegungsumwandlungselement gemäß einer zweiten Ausführungsform der Erfindung.

Fig. 11c ist eine Ansicht von unten auf das Druck-Bewegungsumwandlungselement gemäß einer zweiten Ausführungsform der Erfindung.

Fig. 11d ist Seitenansicht des Druck-Bewegungsumwandlungselements gemäß einer zweiten Ausführungsform der Erfindung.

Fig. 11e ist eine Schnittansicht des Druck-Bewegungsumwandlungselements gemäß einer zweiten Ausführungsform der Erfindung entlang Linie A-A in Fig. 11a.

Fig. 11f ist eine vergrößerte Ansicht des Ausschnitts B in Fig. A-A.

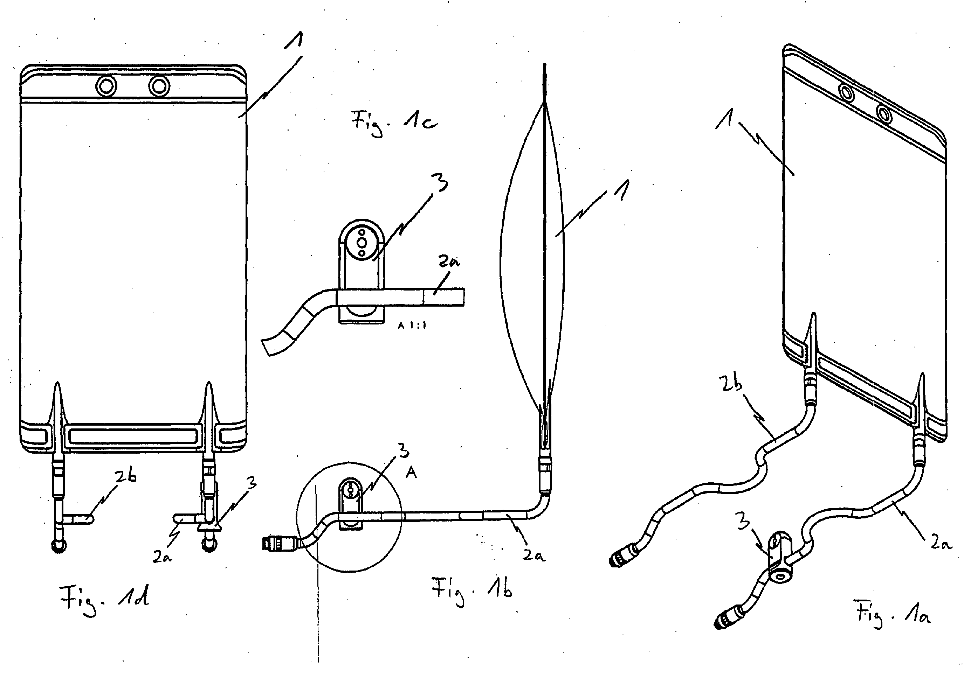

In Fig. 1a, 1b und 1d ist ein Hydraulikfluidbeutel 1 abgebildet, der zur Bevorratung von Schmiermittel, wie Öl, Wasser, eine Öl-Wasser-Emulsion, etc, für eine Endoskopvorrichtung verwendet wird. Der Fluidbeutel 1 weist zwei Verbindungsschläuche 2a, 2b, insbesondere Silikonschläuche, auf, über die das Schmiermittel (vorzugsweise flüssig) bzw. Fluid zu einem Endoskop zugeführt wird. Im Speziellen wird das Fluid aus dem Fluidbeutel 1 beispielsweise mittels einer Kreiselpumpe, in die die Verbindungsschläuche 2a, 2b eingelegt sind und die Fluid in den Verbindungsschläuchen 2a, 2b in Bewegung versetzt, zu den Schmierstellen geführt. Dabei wird z.B. über den Verbindungsschlauch 2b Schmiermittel zu einer Schmierstelle zwischen den Innenseiten eines Stülpschlauchs bzw. ins Innere des Stülpschlauchs eines Endoskops zugeführt, während über den Verbindungsschlauch 2a Schmiermittel zu einer Schmierstelle zwischen dem Endoskopschaft und der an dem Endoskopschaft anliegenden Außenseite des Stülpschlauchs zugeführt wird.

Um den Druck zu erfassen, mit dem Schmiermittel zu der Schmierstelle durch den Schlauch 2a zugeführt wird, ist an dem Schlauch 2a ein Druck-Bewegungsumwandlungselement 3 befestigt, welches zusammen mit einem Kraftmesselement 5, das später beschrieben wird, eine Druckmesseinrichtung der Erfindung bildet.

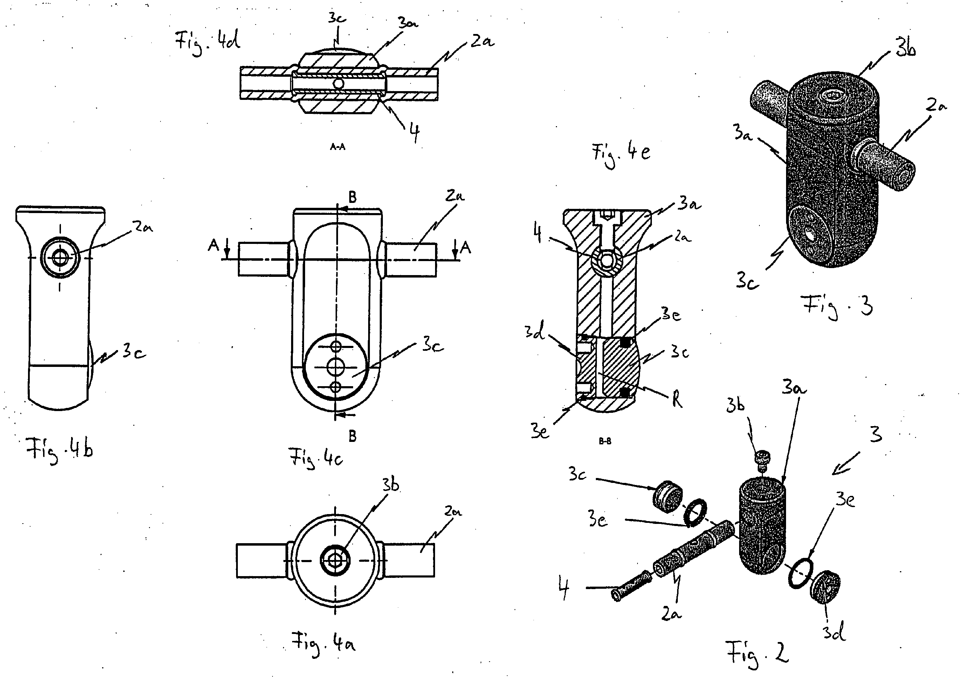

Ein Druck-Bewegungsumwandlungselement 3 gemäß einer ersten Ausführungsform ist in Fig. 2 in einer Explosionsansicht und in Fig. 3 in einer Perspektivenansicht gezeigt. Das Druck-Bewegungsumwandlungselement 3 gemäß der ersten Ausführungsform besteht im Wesentlichen aus einem länglichen Gehäuse 3a mit einer Längsbohrung und zwei zueinander rechtwinklig angeordnet Querbohrungen, wobei die Querbohrungen über die Längsbohrung miteinander verbunden sind, einem Verschlusselement 3b, einem Kolben 3c, einem Stopfen 3d, sowie zwei Dichtungsringen 3e. Gehäuse 3a, Verschlusselement 3b, Kolben 3c sowie Stopfen 3d sind bevorzugt aus Kunststoff und durch Spritzguss herstellbar.

Wie in Fig. 2 und 4e gezeigt, die eine Schnittansicht des Druck-Bewegungsumwandlungselements 3 entlang Linie B-B in Fig. 4c ist, erstreckt sich die Längsbohrung von Gehäuseoberseite durch die obere Querbohrung hindurch, und öffnet in die untere Querbohrung. Die durch die Längsbohrung ausgebildete Öffnung in der Gehäuseoberseite ist mit einem Verschlusselement 3b, das in dieser Ausführungsform als Schraube ausgebildet ist, fluiddicht verschlossen.

Mit Bezug auf Fig. 3 und 4b ist eine Seite der unteren Querbohrung mit dem Stopfen 3d verschlossen, der in das Gehäuse 3a eingepasst bzw. eingeschraubt ist, wobei zwischen Gehäuse 3a und Stopfen 3d eine Dichtung 3f für eine fluiddichte Abdichtung vorgesehen ist. An der anderen Seite der unteren Querbohrung ist ein bewegliches Element 3c fluiddicht eingepasst. Auf diese Weise ist in der unteren Querbohrung zwischen dem Stopfen 3d und dem beweglichen Element 3c ein Druckraum R ausgebildet, wie in Fig. 4e gezeigt ist. Das bewegliche Element 3c ist in dieser Ausführungsform als ein mit einer Dichtung 3f umgebener Kolben 3c ausgebildet, welcher in der unteren Querbohrung axial verschieblich angeordnet ist. Die dem Druckraum R zugewandte Stirnseite des Kolbens 3d ist eine Druckaufnahmefläche. Die dem Druckraum R abgewandte Stirnseite des Kolbens 3d steht bauchig nach außen von der Gehäuseaußenseite vor.

Wie in den Fig. 2, 3, 4a-4e gezeigt ist, stellt die obere Querbohrung einen Aufnahmeabschnitt des Gehäuses 3a dar. Ein Abschnitt des Verbindungsschlauchs 2a ist in die obere Querbohrung eingesetzt bzw. eingepasst, wie in Fig. 4d und 4e gezeigt ist. Dabei ist der Verbindungsschlauch 2a derart in die obere Querbohrung eingepasst, dass er eine fluiddichte Verbindung mit dem Gehäuse 3a ausbildet. Um diese Abdichtung zuverlässig aufrecht zu erhalten und ein partielles Ablösen der Verbindungsschlauchaußenseite von der Innenseite der Querbohrung zu verhindern, ist eine Stützhülse 4 im Inneren des Verbindungsschlauchs 2a angeordnet, die den Verbindungsschlauch 2a gegen die Innenseite der oberen Querbohrung abstützt bzw. drückt. Die Stützhülse 4 ist vorteilhaft aus einem elastischen Kunststoff ausgebildet, um in Einbaulage eine radial nach außen wirkende Kraft auf den Verbindungsschlauch 2a aufzubringen. Wie in Fig. 4d gezeigt ist, ist die Stützhülse 4 an ihren Enden vorteilhaft mit einem Vorsprung versehen bzw. nach außen aufgeweitet, so dass der Verbindungsschlauch in einer vorbestimmten Position bezüglich des Gehäuses 3a fixiert ist. Zusätzlich oder alternativ können der Verbindungsschlauch 2a und die Stützhülse 4 auch mittels des Verschlusselements 3b bezüglich des Gehäuses 3a fixiert werden, z.B. indem das Verschlusselement 3b in Einbaulage gegen die Außenseite des Verbindungsschlauchs 2a drückt.

Wie z.B. in Fig. 4d und 4e gezeigt ist, haben der Verbindungsschlauch 2a und die Stützhülse 4 Öffnungen in ihrer Radialrichtung ausgebildet, die in die Längsbohrung des Gehäuses 3a münden. Somit besteht über die Öffnungen und die Längsbohrung eine Verbindung zwischen dem Inneren des Verbindungsschlauchs 2a und dem Druckraum R.

Die vorstehend beschriebene Verbindung kann vorteilhaft dadurch realisiert werden, dass nach einem Einsetzen des Verbindungsschlauchs 2a zusammen mit der Stützhülle 4 in die obere Querbohrung von der Gehäuseoberseite ausgehend gebohrt wird, um gleichzeitig die Öffnungen in der Stützhülle 4 und dem Verbindungsschlauch 2a sowie die Längsbohrung auszubilden. Auf diese Weise ist sichergestellt, dass die Öffnungen der Stützhülse 4 und des Verbindungsschlauchs 2a mit der Längsbohrung ausgerichtet sind, um eine zuverlässige Verbindung des Inneren des Verbindungsschlauchs 2a dem Druckraum R zu gewährleisten.

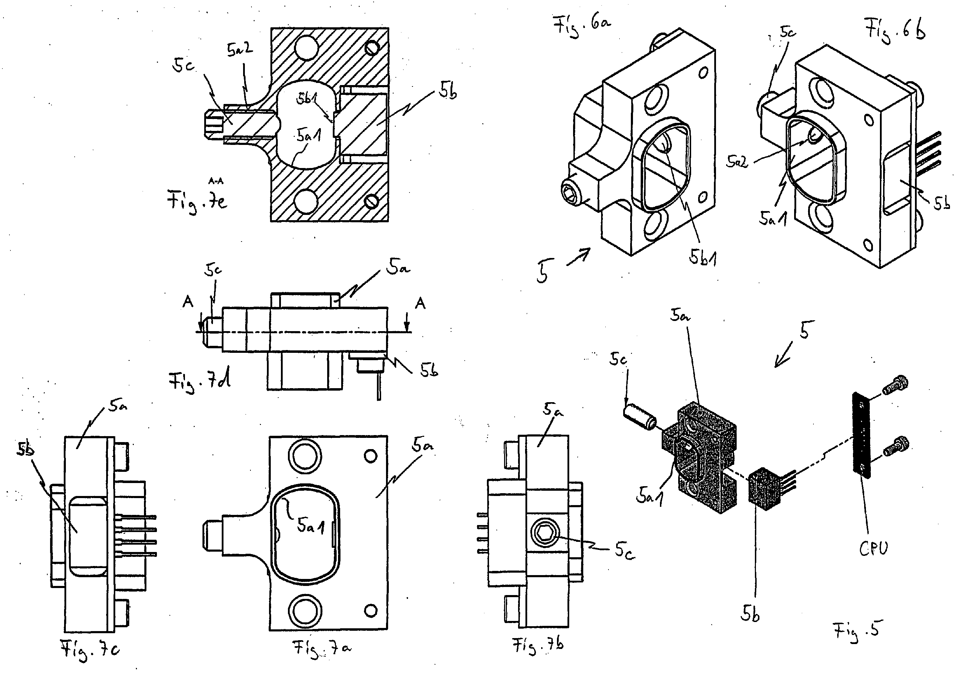

Das vorstehend beschriebene Druck-Bewegungsumwandlungselement 3 wirkt mit einem Kraftmesselement 5 zusammen, um eine Druckmesseinrichtung gemäß der Erfindung auszubilden. Wie in der Fig. 5 gezeigt ist, besteht das Kraftmesselement 5 im Wesentlichen aus einem Gehäuse 5a, einem Kraftmesssensor 5b und einer Positioniereinrichtung 5c. Das Gehäuse hat eine im wesentlichen rechteckige Form mit einem vorspringenden Abschnitt. Wie in Fig. 5 gezeigt ist, ist in der Vorderfläche des Gehäuses eine Öffnung 5a1 ausgebildet, die zu dem unteren Bereich des Druck-Bewegungsumwandlungselements 3 korrespondiert. In der Seitenfläche des vorspringenden Abschnitts ist eine Durchgangsbohrung 5a2 ausgebildet, die in die Öffnung 5a1 mündet. In der Durchgangsbohrung 5a2 ist die Positioniereinrichtung 5c angeordnet. In dieser Ausführungsform besteht die Positioniereinrichtung 5c aus einem federvorgespannten Stift, der durch Federkraft bis zu einem bestimmten Ausmaß ins Innere der Öffnung 5a1 gedrückt wird. Des Weiteren ist in der dem vorspringenden Abschnitt gegenüberliegenden Seite des Gehäuses 5a eine weitere Öffnung 5a3 ausgebildet, welche in die Öffnung 5a1 mündet. Der Kraftmesssensor 5b ist in die Öffnung 5a3 so eingesetzt, dass seine Kraft erfassende Fläche 5b1 im Inneren der Öffnung 5a1 frei und der Positioniereinrichtung 5c gegenüber liegt, wie in Fig. 7e gezeigt ist. Der Kraftmesssensor 5b ist in dieser Ausführungsform ein Sensor, der eine auf seine Kraft erfassende Fläche 5b1 einwirkende Kraft in elektrische Signale umwandelt, die dann über mittels einer CPU, wie in Fig. 5 gezeigt, oder ähnlichem ausgewertet werden können. Alternativ kann jedoch auch ein mechanischer Kraftmesssensor verwendet werden.

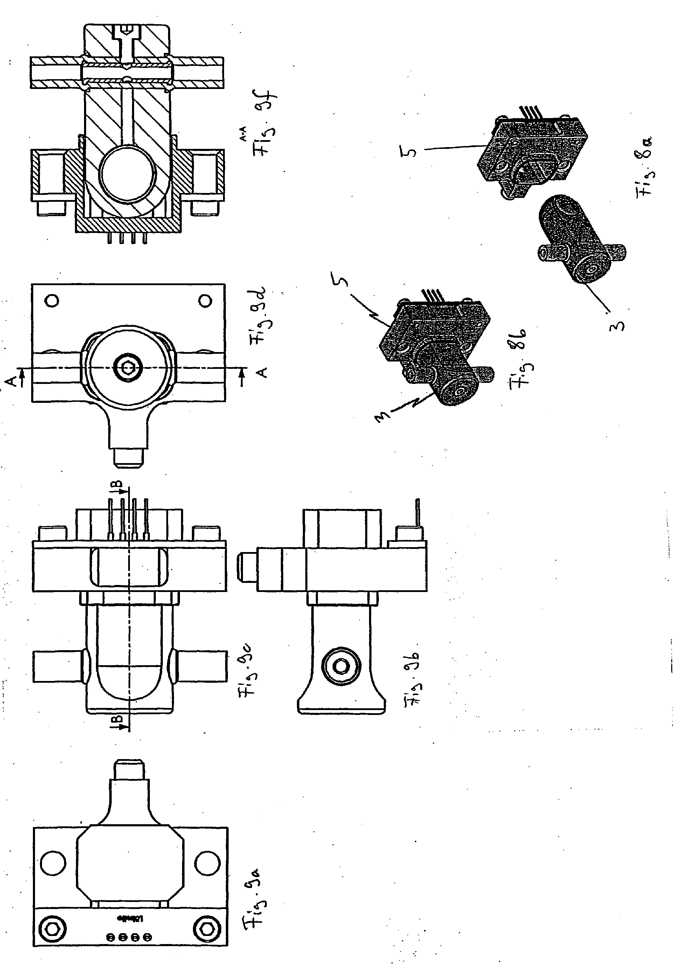

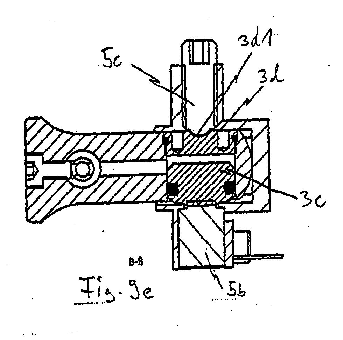

Fig. 8b, 9a-9f zeigen die Druckmesseinrichtung der Erfindung in zusammengestecktem Zustand in verschiedenen Ansichten. Wie aus der Querschnittansicht von Fig. 9e erkennbar ist, drückt in dem zusammengesteckten Zustand der federvorgespannte Stift 5c mit seinem bauchigen Ende gegen eine in dem Stopfen 3d ausgebildete und zu der bauchigen Form des Stiftendes korrespondierende Aussparung 3d1, um das Druck-Bewegungsumwandlungselement 3 in Richtung des Kraftmesssensor 5b zu drücken, so dass das Druck-Bewegungsumwandlungselement 3 in der Öffnung 5a1 des Kraftmesselements 5 in einer vorbestimmten Position angeordnet ist. Genauer gesagt ist die Position derart, dass die bauchige Kolbenaußenfläche an der Kraft erfassenden Fläche 5b1 des Kraftmesssensors 5b anliegt. Bevorzugt ist das Druck-Bewegungsumwandlungselement 3 mittels der Positioniereinrichtung 5c derart fixiert, dass sich ihr Gehäuse 3a auch bei Bewegung des Kolbens 3c nicht relativ zu dem Gehäuse 5a des Kraftmesselements 5 bewegt. Darüber hinaus ist der federvorgespannte Stift vorzugsweise so ausgelegt, dass das Druck-Bewegungselement 3 problemlos von Hand in das Kraftmesselement 5 eingesetzt und wieder herausgezogen werden kann.

Zusätzlich oder alternativ kann entweder das Druck-Bewegungsumwandlungselement 3 oder das Kraftmesselement 5 mit einer Arretiervorrichtung versehen sein, die ein Trennen des Druck-Bewegungsumwandlungselements 3 und des Kraftmesselements 5 in dem zusammengesteckten Zustand verhindert. Eine derartige Arretiervorrichtung kann bspw. ein an dem Kraftmesselement 5 befestigter und in eine Arretierposition bringbarer Stift sein, der in zusammengesteckten Zustand der Druckmesseinrichtung in eine in dem Druck-Bewegungsumwandlungselement 3 ausgebildete Aussparung eingreift, derart, dass in Arretierposition das Druck-Bewegungsumwandlungselement 3 nicht mehr von Hand aus dem Kraftmesselement 5 herausgezogen werden kann.

Nachfolgend wird die Verwendung bzw. Funktionsweise der Druckmesseinrichtung gemäß der Erfindung mit Bezug auf eine Einwegendoskopvorrichtung beschrieben.

Vor einer Inbetriebnahme der Druckmesseinrichtung wird das Druck-Bewegungsumwandlungselement 3 mit dem Verbindungsschlauch 2a des Fluidbeutels 2 verbunden bzw. ist an diesem vormontiert, wie vorstehend mit Bezug auf Fig. 1a-1d, 2, 3, 4a-4e beschrieben ist.

Der Verbindungsschlauch 2a wird mit einem Endoskop verbunden, um Schmierflüssigkeit von dem Fluidbeutel 2 zu der Schmierstelle zwischen Endoskopschaft und der daran anliegenden Stülpschlauchaußenseite zu liefern. Dazu wird der Verbindungsschlauch an eine Kreiselpumpe oder Ähnliches angeschlossen, die das Fluid in dem Verbindungsschlauch durch Außeneinwirkung auf den Verbindungsschlauch 2a mit einem gewissen Druck zu der Schmierstelle fördert.

Um den Druck des zugeführten Schmiermittels zu messen, wird vor Druckbeaufschlagung des Fluids in dem Verbindungsschlauch 2a mittels der Kreiselpumpe das mit dem Verbindungsschlauch 2a verbundene Druck-Bewegungsumwandlungselement 3 in die Öffnung 5a1 des Kraftmesselements 5 eingesteckt, um auf diese Weise den zusammengesteckten Zustand, der in Fig. 8b, 9a-9f gezeigt ist, und damit den funktionsfähigen Zustand der Druckmesseinrichtung der Erfindung zu erreichen. In diesem Zustand greift das bauchige Ende des federvorgespannten Stifts 5c in die Aussparung des Stopfens 3d1 und drückt das Druck-Bewegungsumwandlungselement 3 in Inneren der Öffnung 5a1 in Richtung des Kraftmesssensors 5b derart, dass die bauchige Kolbenaußenseite des Kolbens 3c an der Kraft aufnehmenden Fläche 5b1 des Kraftmesssensors 5b anliegt.

Das Innere des Verbindungsschlauchs 2a ist durch die Öffnungen in dem Verbindungsschlauch 2a und der Stützhülse 4, die obere Querbohrung und die Längsbohrung mit der Druckkammer R fluidverbunden. Somit befindet sich in der Druckkammer R Fluid, das den gleichen Druck hat, wie Fluid in dem Verbindungsschlauch 2a bzw. dem Fluidbehälter 1. Da eine Druckbeaufschlagung mittels der Kreiselpumpe noch nicht stattgefunden hat, wird in diesem Zustand von einem Ruhedruck gesprochen. Das Fluid mit Ruhedruck in der Druckkammer R führt dazu, dass der Kolben 3c mit einer gewissen Kraft in Richtung des Kraftmesssensors 5b gedrängt wird, so dass der Kraftmesssensor 5b eine über seine Druck aufnehmende Fläche 5b1 eine dem Ruhedruck entsprechende Kraft registriert. Der Kraftmesssensor wird nun gemäß dieser Kraft kalibriert, d.h. auf Null eingestellt.

Anschließend wird das Fluid in dem Verbindungsschlauch durch Bewegung der Kreiselpumpe druckbeaufschlagt. Das führt zu einer Druckänderung des Fluids in dem Verbindungsschlauch 2a und der Druckkammer R. Aufgrund dieser Druckänderung ändert sich die auf die Druckaufnahmefläche des Kolbens 3c einwirkende Kraft, die wiederum zu einer Bewegung des Kolbens 3c in Richtung des Kraftmesssensors 5b führt. Die Bewegung des Kolbens 3c verursacht einen Anstieg der auf die Kraft erfassende Fläche 5b1 des Kraftmesssensors 5b wirkenden Kraft. Die auf den Kraftmesssensor 5b wirkende Kraft wird erfasst, und in Form von elektrischen Signalen an eine Auswerteeinheit, beispielsweise eine CPU, übermittelt. Die CPU kann von der durch den Kraftmesssensor 5b gemessenen Kraft auf einen Druck des Fluids in dem Verbindungsschlauch 2a rückschließen bzw. den Druck des Fluids von dieser Kraft ableiten, und auf diese Weise den Druck des Fluids durch Steuerung der Kreiselpumpen auf Basis der erfassten Werte auf den gewünschten Wert einstellen.

Nach Abschluss einer Untersuchung mit der Endoskopvorrichtung, die eine Einwegendoskopvorrichtung ist, wird das Druck-Bewegungsumwandlungselement 3 durch Herausziehen aus der Öffnung 5a1 von dem Kraftmesselement 5 getrennt, wobei zumindest das Kraftmesselement 3, bevorzugt aber auch das Druck-Bewegungsumwandlungselement 3, nicht beschädigt werden. Das Druck-Bewegungsumwandlungselement 3 kann dann zusammen mit dem Endoskopschaft samt Stülpschlauch, dem Fluidbeutel 1 und allen Verbindungsleitungen von dem Fluidbeutel zu der Schmierstelle, welche mit Körperflüssigkeit des untersuchten Patienten kontaminiert sein könnten, entsorgt werden, während das Kraftmesselement 5, das aufgrund der fluiddichten Ausbildung des Druck-Bewegungsumwandlungselement 3 nicht mit dem Schmiermittel bzw. Körperflüssigkeit in Kontakt gekommen ist, wieder verwendet werden kann.

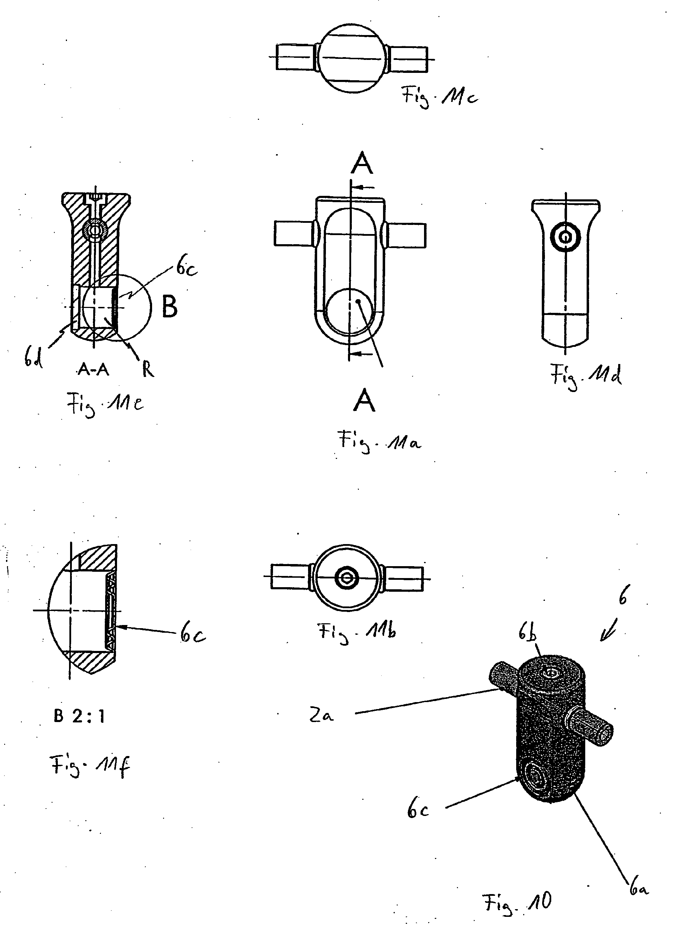

Fig. 10, 11a bis 11f zeigen eine zweite Ausführungsform des Druck-Bewegungsumwandlungselements gemäß der Erfindung. Das Druck-Bewegungsumwandlungselement 6 gemäß der zweiten Ausführungsform hat einen ähnlichen Aufbau wie das Druck-Bewegungsumwandlungselement 3 der ersten Ausführungsform, und nachstehend werden nur die Unterschiede zu dieser ersten Ausführungsform beschrieben.

Im Gegensatz zu der dem Druck-Bewegungsumwandlungselement 3 der ersten Ausführungsform weist das Druck-Bewegungsumwandlungselement 6 gemäß der zweiten Ausführungsform anstelle des Kolbens 3c und des Stopfens 3d eine Membran 6c und einen Deckel 6d auf. Die Membran 6c ist vorzugsweise aus einen Kautschukmaterial ausgebildet und ist in die untere Querbohrung des Gehäuses 6a fluiddicht eingepasst, vorzugsweise eingeklebt, wie in Fig. 11e und 11f gezeigt ist. Die Membran 6c ist funktionsgleich mit dem Kolben 3c, d.h. sie ist dergestalt, dass sie sich bei einer Druckerhöhung in der Druckkammer R nach außen auswölbt, und somit eine Kraft auf einen Kraftmesssensor 5b ausüben kann.

Im Gegensatz zu dem Stopfen 3d, der mit einer Dichtung 3f versehen und in das Gehäuse 3a des Druck-Bewegungsumwandlungselement 3 der ersten Ausführungsform eingeschraubt ist, ist der Deckel 6d fluiddicht eingeklebt.

Das Druck-Bewegungsumwandlungselement 6 der zweiten Ausführungsform wirkt mit dem Kraftmesselement 5 in gleicher Weise wie das Druck-Bewegungsumwandlungselement 3 gemäß der ersten Ausführungsform zusammen.

Obwohl die vorliegende Erfindung mit Bezug auf einen Schlauchabschnitt des Verbindungsschlauchs 2a des Hydraulikbeutels beschrieben worden ist, kann das Druck-Bewegungsumwandlungselement mit einem anderen Element der Schmiermittelzuführung verbunden bzw. fluidverbunden sein. In dieser Hinsicht kann das Druck-Bewegungsumwandlungselement auch mit einem Verbindungsschlauchstück verbunden bzw. fluidverbunden sein, das mit Elementen der Schmiermittelzuführung verbunden wird, wie z.B. zwei Schmiermittelzuführschläuchen.

Eine Druckmesseinrichtung besteht aus zwei getrennten Bauteilen, die von Hand zusammensteckbar und wieder lösbar sind. Das eine Bauteil ist ein Druck-Bewegungsumwandlungselement (3, 6), das einen Druck aufnimmt und in dessen Abhängigkeit eine Bewegung eines beweglichen Abschnitts (3c, 6c) von sich bewirkt, und das andere Bauteil ist ein Kraftmesselement (5), welches eine Kraft misst, die auf Grund der Bewegung des beweglichen Abschnitts (3c, 6c) verursacht wird. Das Druck-Bewegungsumwandlungselement (3, 6) ist als Einwegbauteil konstruiert, während das Kraftmesselement (5) zur Wiederverwendung bestimmt ist.

## Patentansprüche

1. Druckmesseinrichtung für Einwegendoskopievorrichtungen bestehend aus einem Druck-Bewegungsumwandlungselement (3, 6) mit einem beweglichen Abschnitt (3c, 6c) als ein Kolben oder eine Membran, welches einen Fluiddruck aufnimmt und in Abhängigkeit von dessen Änderung eine Bewegung des beweglichen Abschnitts (3c, 6c) bewirkt, und einem Kraftmesselement (5), welches eine Kraft misst, die durch die Bewegung des beweglichen Abschnitts (3c, 6c) darauf ausgeübt wird, wobei das Druck-Bewegungsumwandlungselement (3, 6) und das Kraftmesselement (5) als zusammensteckbare fluiddicht getrennt, mechanisch koppelbare, vorzugsweise separate Bauteile ausgebildet sind, wobei
das Kraftmesselement (5) ein Gehäuse (5a) mit Öffnung (5a1) aufweist, **dadurch gekennzeichnet, daß**
in einem Innenraum des Gehäuses (5a) sich gegenüberliegend ein Kraftmesssensor (5b) und eine Positioniereinrichtung (5c) zum Positionieren des Druck-Bewegungsumwandlungselements (3, 6) in einer vorbestimmten Lage relativ zu dem Kraftmesssensor (5b) angeordnet sind, und
in Funktionszustand der Druckmesseinrichtung das Druck-Bewegungsumwandlungselement (3, 6) mit dem beweglichen Abschnitt (3c, 6c) über die Öffnung (5a1) derart in das Gehäuse (5a) eingesetzt ist, dass es zwischen dem Kraftmesssensor (5b) und der Positioniereinrichtung (5c) fixiert ist, wobei der bewegliche Abschnitt (3c, 6c) an dem Kraftmesssensor (5b) anliegt.

2. Druckmesseinrichtung gemäß Patentanspruch 1, wobei das Druck-Bewegungsumwandlungselement (3, 6) als eine gas- bzw. fluiddichte Einheit ausgebildet ist.

3. Druckmesseinrichtung gemäß einem der Patentansprüche 1 oder 2, wobei das Druck-Bewegungsumwandlungselement (3, 6) einen Aufnahmeabschnitt zur Aufnahme eines Abschnittes Behältnisses oder Schlauchs (2a) hat, durch welchen ein druckbeaufschlagtes Gas oder Fluid strömt, dessen Druck durch die Druckmesseinrichtung gemessen werden soll.

4. Druckmesseinrichtung gemäß Patentanspruch 3, des Weiteren mit einem Einwegfluidbeutel (1), welcher ein Fluid bevorratet, wobei der Aufnahmeabschnitt einen Schlauchabschnitt eines Schlauchs (2a) aufnimmt, der mit dem Einwegfluidbeutel (1) verbundenen ist.

5. Druckmesseinrichtung gemäß einem der Ansprüche 1 bis 4, wobei das Kraftmesselement (5) oder das Druck-Bewegungsumwandlungselement (3, 6) mit einer Arretierungseinrichtung versehen ist, um das Druck-Bewegungsumwandlungselement (3, 6) in dem zusammengesteckten Zustand relativ zu dem Kraftmesselement (5) zu fixieren.

## Claims

1. A pressure gauge for one-way endoscopic devices comprising:
a pressure-motion conversion element (3, 6) having a movable portion (3c, 6c) in form of a piston or membrane for receiving a fluid pressure and effecting a movement of the movable portion in response to the change thereof; and
a force measuring element (5) measuring a force exerted thereon by the movement of the movable portion (3c, 6c);
wherein the pressure-motion conversion element (3, 6) and the force measuring element (5) are formed as preferably separate components that can be pieced together, are separated in a fluid-tight manner, and can be coupled mechanically; wherein the force measuring element (5) comprises:
a housing (5a) with an orifice (5a1); **characterized in that** within said housing (5a)
a force measuring sensor (5b), and a positioning means (5c) are arranged opposite to each other for positioning the pressure motion conversion element (3, 6) in a predetermined position relative to said force measuring sensor (5b),
wherein the pressure motion conversion element (3, 6) is inserted with its moveable portion (3c, 6c) through the orifice (5a1) into a fixed position between the force measuring sensor (5b) and the positioning means (5c) in the housing (5a) when the pressure gauge is in a operative state, wherein said movable portion (3c, 6c) is in contact with said force measuring sensor (5b).

2. The pressure gauge of claim 1, wherein
the pressure-motion conversion element (3, 6) is formed as a gas- or fluid-tight unit.

3. The pressure gauge of claim 1 or 2, wherein:
the pressure-motion conversion element (3, 6) has a receiving portion for receiving a portion of a container or tube (2a) through which a pressurized gas or fluid flows, whose pressure is to be measured by the pressure gauge.

4. The pressure gauge of claim 3, **characterized by** a disposable fluid bag (1) storing a fluid,
wherein the receiving portion receives a tube portion of a tube (2a), which is connected to said disposable fluid bag (1).

5. The pressure gauge of one of claims 1 to 4, wherein:
the force measuring element (5) or the pressure-motion conversion element (3, 6) comprises
a locking mechanism for fixing the pressure-motion conversion element (3, 6) in an assembled state thereof relative to the force measuring element (5).

## Revendications

1. Dispositif de mesure de pression pour des endoscopes à usage unique, constitué d'un élément (3, 6) de transformation de pression en déplacement, avec une partie mobile (3c, 6c) sous la forme d'un piston ou d'une membrane, qui reçoit une pression de fluide et produit en fonction de sa modification un déplacement de la partie mobile (3c, 6c), et d'un élément (5) de mesure de force qui mesure une force qui est exercée par le déplacement de la partie mobile (3c, 6c) sur cet élément, sachant que l'élément (3, 6) de transformation de pression en déplacement et l'élément (5) de mesure de force sont réalisés sous la forme d'éléments de préférence séparés, mécaniquement accouplables et pouvant être assemblés par emboîtement en étant isolés en étanchéité aux fluides,
sachant que l'élément (5) de mesure de force présente un boîtier (5a) doté d'une ouverture (5a1), **caractérisé en ce que**
un capteur (5b) de mesure de force et un dispositif de positionnement (5c) pour positionner l'élément (3, 6) de transformation de pression en déplacement dans une position prédéterminée par rapport au capteur (5b) de mesure de force sont disposés en vis-à-vis dans un espace intérieur du boîtier (5a),
et
dans l'état fonctionnel du dispositif de mesure de pression, l'élément (3, 6) de transformation de pression en déplacement est inséré par sa partie mobile (3c, 6c), via l'ouverture (5a1), dans le boîtier (5a) de telle sorte qu'il est immobilisé entre le capteur (5b) de mesure de force et le dispositif de positionnement (5c), la partie mobile (3c, 6c) s'appliquant contre le capteur (5b) de mesure de force.

2. Dispositif de mesure de pression selon la revendication 1, sachant que l'élément (3, 6) de transformation de pression en déplacement est réalisé sous la forme d'une unité étanche aux gaz et/ ou aux fluides.

3. Dispositif de mesure de pression selon la revendication 1 ou 2, sachant que l'élément (3, 6) de transformation de pression en déplacement possède une partie réceptrice pour recevoir une partie d'un récipient ou d'un tuyau souple (2a) à travers laquelle s'écoule un gaz ou fluide sollicité en pression dont la pression doit être mesurée par le dispositif de mesure de pression.

4. Dispositif de mesure de pression selon la revendication 3, avec en outre une pochette (1) pour fluide à usage unique qui met en réserve un fluide, sachant que la partie réceptrice reçoit une partie d'un tuyau souple (2a) qui est reliée à la pochette (1) pour fluide à usage unique.

5. Dispositif de mesure de pression selon l'une des revendications 1 à 4, sachant que l'élément (5) de mesure de force ou l'élément (3, 6) de transformation de pression en déplacement est pourvu d'un dispositif de blocage pour immobiliser l'élément (3, 6) de transformation de pression en déplacement dans l'état assemblé par emboîtement par rapport à l'élément (5) de mesure de force.
